(19) **Europäisches Patentamt European Patent Office Office européen des brevets**

(11) **EP 4 370 202 B1**

(12) **EUROPEAN PATENT SPECIFICATION**

(45) Date of publication and mention of the grant of the patent:
**18.03.2026 Bulletin 2026/12**

(21) Application number: **22744147.4**

(22) Date of filing: **01.07.2022**

(51) International Patent Classification (IPC):
*A61N 5/10* (2006.01)    *A61K 41/00* (2020.01)
*A61P 35/00* (2006.01)    *A61K 31/13* (2006.01)
*A61K 33/00* (2006.01)    *A61P 25/00* (2006.01)

(52) Cooperative Patent Classification (CPC):
(C-Sets available)
**A61N 5/10; A61K 31/13; A61K 33/00; A61K 41/0038; A61P 25/00; A61P 35/00; A61N 2005/1098**    (Cont.)

(86) International application number:
**PCT/EP2022/068289**

(87) International publication number:
**WO 2023/285170 (19.01.2023 Gazette 2023/03)**

(54) **XENON GAS FOR USE IN THE TREATMENT OF GLIOMAS**

**XENONGAS ZUR VERWENDUNG BEI DER BEHANDLUNG VON GLIOMEN**

**XÉNON GAZEUX DESTINÉ À ÊTRE UTILISÉ DANS LE TRAITEMENT DE GLIOMES**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.07.2021 EP 21184988**

(43) Date of publication of application:
**22.05.2024 Bulletin 2024/21**

(73) Proprietor: **Universität Heidelberg**
**69177 Heidelberg (DE)**

(72) Inventors:
 • **RUDER, Arne Mathias**
   **68167 Mannheim (DE)**
 • **GIORDANO, Frank A.**
   **53127 Bonn (DE)**

(74) Representative: **Müller-Boré & Partner**
**Patentanwälte PartG mbB**
**Friedenheimer Brücke 21**
**80639 München (DE)**

(56) References cited:
**US-A- 4 820 258**    **US-A1- 2017 341 980**

 • **A. L. RIVERA ET AL: "MGMT promoter methylation is predictive of response to radiotherapy and prognostic in the absence of adjuvant alkylating chemotherapy for glioblastoma", NEURO-ONCOLOGY, vol. 12, no. 2, 14 December 2009 (2009-12-14), US, pages 116 - 121, XP055614619, ISSN: 1522-8517, DOI: 10.1093/neuonc/nop020**

(52) Cooperative Patent Classification (CPC): (Cont.)

C-Sets
**A61K 31/13, A61K 2300/00;**
**A61K 33/00, A61K 2300/00**

**Description**

The present invention relates to xenon gas for use in *in vivo* methods of sensitizing glioma cells in a subject for radiation therapy, as well as related *in vitro* methods. Document US 2017/341980 A1 discloses application of Xenon gas for sensitizing glioma cells for radiation therapy, wherein the gas is administered to the patient after the radiation therapy.

Brain tumors, so-called gliomas, are diagnosed as a total entity in approximately 5 out of 100,000 people per year. A classification is made histopathologically into WHO grades 1 to 4, wherein WHO grades 3 and 4 are called high-grade gliomas and represent malignant tumors. Glioblastoma (WHO grade 4) is considered the most common glioma in adults. The prognosis is unfavorable, with a median survival expectancy of 14 months from diagnosis despite maximal therapy. High-grade glioma disease can occur at any age, with frequency peaks appearing between 35 and 44 years of age, and between 75 and 84 years of age.

For more than 15 years, the standard therapy for high-grade gliomas has been the triple approach of surgical resection followed by radiation chemotherapy with an alkylating agent (e.g., temozolomide) and subsequent continuation of alkylating chemotherapy alone. Receiving standard therapy, patients with high-grade gliomas and/or gliomas having a methylated promoter of the $O^6$-methylguanine-DNA methyltransferase (MGMT) gene show improved median survival with over 23 months. Yet, for patients with unmethylated MGMT promoters, representing more than half of all high-grade glioma patients, the addition of chemotherapy yields almost no effect in prolonging median overall survival compared to radiotherapy alone. Therefore, high grade gliomas with unmethylated MGMT promoters are being increasingly treated in clinical trials omitting alkylating chemotherapy and combining radiotherapy with experimental drugs.

Since high-grade gliomas exhibit infiltrative growth, but surgery with a safe distance to the tumor in the brain is not possible, tumor cells remaining postoperatively are to be eliminated by subsequent treatment with ionizing radiation (usually photon beam radiation). In this case, the therapy is usually performed as an outpatient treatment series: A radiation dose of 2 Gy is applied daily to the affected area of the brain until a total dose of 60 Gy is reached after 30 irradiations. The radiation causes damage to the tumor cell DNA, which, if not repaired promptly, leads to the demise of the tumor cell. Generally, cell-internal repair processes start within a few minutes and are mostly completed after 120 minutes. However, the effect of radiation treatment is limited by the high DNA repair capacity of glioma cells, which are therefore considered resistant to radiation. So far, limited success of the current multimodal therapy is likely due to the diffuse nature of the tumor and this prominent resistance to ionizing radiation with a high degree of recurrent invasive growth of the glioblastoma cells after the treatment. Addition of chemotherapy to radiation treatment is a way to decrease radioresistance, but in glioblastoma with overexpression of MGMT due to an unmethylated promoter region, standard alkylating chemotherapy (using e.g., temozolomide) is antagonized by MGMT-mediated removal of temozolomide's adducts at the $O^6$-position of guanine at DNA level. In view of this and the outstanding role of radiation therapy for the prognosis of patients with unmethylated MGMT promoters, radiosensitizing substances addressing both, the high inherent migratory and invasive behavior and the intrinsic DNA double-strand break (DSB) repair capacity upon ionizing radiation of glioblastoma cells become increasingly important to improve therapy and patient survival. Thus, the search for a method or a drug for radiosensitization of glioma cells that makes the cells more sensitive to radiation treatment independently of MGMT promoter methylation status is ongoing.

In the past, the tumor growth-promoting effect of special ion channels, so-called alpha-amino-3-hydroxy-5-methyl-4-isoxazole propionic acid receptors (AMPAR) and N-methyl-D-aspartate receptors (NMDAR), could be demonstrated on high-grade gliomas in cell and mouse models, unrelated to their MGMT promoter methylation status. The tumor cells stimulate themselves to proliferate via these ion channels. In addition, AMPAR and NMDAR control metabolic processes, most importantly including DNA damage repair. Studies have successfully inhibited the repair of radiation-induced DNA damage by blocking AMPAR and NMDAR with substances from the class of so-called AMPAR or NMDAR antagonists (such as e.g. talampanel, riluzole, dizocilpine, ifenprodil and memantine).

Most experimentally applied AMPAR and NMDAR antagonists completely block the ion channels. However, healthy brain neurons depend on functioning ion channels, so complete blockade of these receptors causes severe side effects. Therefore, the use of such AMPAR and NMDAR antagonists in humans is not possible. Some of the agents (e.g. memantine, talampanel, perampanel, and riluzole) cause only partial channel blockade and can be administered to humans. However, the accumulation of these agents in brain tissue is insufficient and an effective dose in cell or animal models is not achieved in human brain tissue.

Accordingly, the technical problem underlying the present invention is the provision of new means for the treatment of gliomas, in particular gliomas having an unmethylated MGMT promoter, by sensitizing glioma cells to radiation therapy.

The solution to the above technical problem is achieved by the embodiments characterized in the claims.

In particular, in a first aspect, the present invention relates to xenon gas for use in an *in vivo* method of sensitizing glioma cells in a subject for radiation therapy, wherein the xenon gas is administered to the subject after said radiation therapy, and wherein the glioma cells are glioma cells having an unmethylated promoter of the $O^6$-methylguanine-DNA methyltransferase (MGMT) gene (*i.e.,* having an unmethylated MGMT promoter).

Xenon is a chemical element with the symbol Xe and atomic number 54. It is a colorless, dense, odorless noble gas found in Earth's atmosphere in trace amounts. Naturally occurring xenon consists of seven stable isotopes and two long-lived radioactive isotopes. As used herein, the term "xenon gas" encompasses all of these seven stable isotopes.

The term "glioma cells" as used herein encompasses any tumor cells that are derived from glial cells of the brain or the spine. Preferably, glioma cells in the sense of the present invention are high-grade glioma cells, *i.e.,* cells of WHO grade 3 or grade 4 gliomas, e.g. glioblastoma cells. Preferably, the glioma cells are glioma cells having an unmethylated MGMT promoter.

The term "sensitizing glioma cells for radiation therapy" relates to the process of decreasing said cells' inherent resistance to radiation.

In specific embodiments, the subject does not undergo concurrent chemotherapy. Preferably, the subject does not undergo concurrent chemotherapy with an alkylating agent (i.e., alkylating chemotherapy). As used herein, the term "the subject does not undergo concurrent chemotherapy" indicates that the subject does not receive any such chemotherapy concurrently with the radiation therapy, preferably during the entire period of the radiation treatment regimen.

According to the present invention, the xenon gas is administered to the subject after the radiation therapy, *i.e.,* after completion of said radiation therapy. In this context, the term "after completion of the radiation therapy" as used herein does not refer to completion of radiation therapy as a whole radiation treatment regimen, but to the completion of an individual radiation treatment session.

In preferred embodiments, administration of the xenon gas to the subject starts at the latest 360 minutes after completion of said radiation therapy, more preferably at the latest after 330 minutes, 300 minutes, 270 minutes, 240 minutes, 210, minutes, 180 minutes, 150 minutes, 120 minutes, 105 minutes, 90 minutes, 75 minutes, 60, minutes, 50 minutes, 40 minutes, 30 minutes, 25 minutes, 20 minutes, 15 minutes, 10 minutes, 9 minutes, 8 minutes, 7 minutes, 6 minutes, 5 minutes, 4 minutes, 3 minutes, 2 minutes or 1 minute after completion of said radiation therapy, *i.e.,* after completion of the individual radiation treatment session. Most preferably, administration of the xenon gas to the subject starts immediately, *i.e.,* within 1 minute, after completion of said radiation therapy, *i.e.,* after completion of the individual radiation treatment session.

Preferably, administration of the xenon gas to the subject continues for at least 30 minutes, at least 60 minutes, at least 90 minutes, at least 120 minutes, at least 150 minutes, at least 180 minutes, at least 210 minutes, at least 240 minutes, at least 270 minutes, at least 300 minutes, at least 330 minutes, or for 360 minutes, or any amount of time in between these values. Most preferably, administration of the xenon gas to the subject continues for about 360 minutes, for 330 to 390 minutes, or for any amount of time in between 330 to 390 minutes.

Preferably, the xenon gas is administered to the subject by mouth inhalation and/or nasal inhalation, e.g. using a standard respiratory mask or helmet, or a chamber containing the whole subject, e.g. a hypo- or hyperbaric chamber, as known in the art. Xenon gas administration can be passive, e.g. with inhalation as part of the subjects natural breathing, or active, using non-invasive ventilation techniques with or without a constant positive airway pressure, e.g. non-invasive mask respiration, as known in the art.

According to the present invention, the xenon gas is administered to the subject in a mixture of xenon gas and at least one further breathable gas passively, e.g. with inhalation as part of the subjects natural breathing, or actively, using non-invasive ventilation techniques with or without a constant positive airway pressure.

In this context, further breathable gases in a respective mixture can be selected from e.g. oxygen, nitrogen, carbon dioxide, helium and mixtures thereof. Further breathable gases known in the art are also suitable in the context of the present invention. In specific embodiments, a suitable gas mixture can comprise 1 to 95 % xenon gas (all percentages referring to percent by volume), preferably 20 to 50 % xenon gas; 5 to 99 % oxygen, preferably 15 to 25 % oxygen, e.g. about 20 % oxygen; 0 % to 94 % nitrogen, preferably 30 to 60 % nitrogen; and/or 0 % to 10 % carbon dioxide, preferably 0 % carbon dioxide.

Xenon treatment as in the present invention should be administered with a gas pressure that is about equal to the atmospheric gas pressure at sea level, but could be administered with gas pressure lower than the atmospheric gas pressure at sea level or higher than the atmospheric gas pressure at sea level, e.g. the treatment would be, preferably, administered with a gas pressure of about 1000 hPa, but could be administered with gas pressures between 500 hPa and 10000 hPa.

Radiation therapy in the sense of the present invention is preferably radiation therapy using ionizing radiation as known in the art, preferably wherein the ionizing radiation is selected from the group consisting of photon beam radiation, electron beam radiation, proton beam radiation, and heavy ion beam radiation, more preferably wherein the ionizing radiation is photon beam radiation. In the present innovation, the ionizing radiation can be applied as teletherapy and/or brachytherapy, e.g. with the source of ionizing radiation outside of the subject and/or inside of the subject, respectively.

In specific embodiments, xenon treatment according to the present invention is combined with AMPAR antagonist and/or NMDAR antagonist treatment. Accordingly, in such embodiments, the subject undergoes concurrent therapy with an AMPAR antagonist and/or an NMDAR antagonist. Preferably, the AMPAR antagonist and/or NMDAR antagonist is selected from the group, consisting of the AMPAR antagonists talampanel, perampanel, tezampanel, selurampanel, and GYKI-52466; the AMPAR and NMDAR antagonist kaitocephalin; and the NMDAR antagonists memantine, nitromemantine, neramexane, atomoxetine, amantadine, riluzole, dizocilpine, and ifenprodil. More preferably, the subject undergoes concurrent therapy with the NMDAR antagonist memantine.

In this context, the term "the subject undergoes concurrent therapy with an AMPAR antagonist and/or an NMDAR antagonist" does not require concurrent administration of the AMPAR antagonist and/or NMDAR antagonist with the xenon gas, but refers to the situation wherein the subject undergoing a radiation therapy regimen and xenon gas administration after the individual radiation treatment sessions in accordance with the present invention, additionally receives the AMPAR antagonist and/or NMDAR antagonist over the course of said radiation therapy regimen. Suitable modes of administration, dosages, and administration regimens for the AMPAR antagonist and/or NMDAR antagonist are known in the art.

In a related second aspect, the present invention relates to respective *in vitro* methods, *i.e.,* a method of sensitizing glioma cells for radiation *in vitro,* comprising the step of administering xenon gas to said glioma cells after said glioma cells have been exposed to said radiation, wherein the glioma cells are glioma cells having an unmethylated promoter of the $O^6$-methylguanine-DNA methyl-transferase (MGMT) gene.

In this aspect, all relevant definitions, limitations, and embodiments as defined for the first aspect of present invention apply in an analogous manner. In particular, the glioma cells and types of radiation are as defined above.

As used herein, the term "comprising"/"comprises" expressly includes the terms "consisting essentially of"/"-consists essentially of" and "consisting of"/"consists of", *i.e.,* all of said terms are interchangeable with each other herein.

Further, as used herein, the term "about" preferably represents a modifier of the specified value of $\pm$ 10%, more preferably $\pm$ 10%, $\pm$ 8%, $\pm$ 6%, $\pm$ 5%, $\pm$ 4%, $\pm$ 3%, $\pm$ 2%, $\pm$ 1%, or $\pm$ 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention provides xenon gas for use in sensitizing glioma cells in a subject for radiation therapy.

The inert gas xenon has been used as an anesthetic gas since 1952. It is considered to have particularly few side effects and is therefore used today primarily for long surgical procedures with particularly high cardiovascular stress. Xenon gas has a pronounced neuroprotective effect and can prevent nerve cells in the brain from dying in the event of a lack of oxygen. Because of this, xenon gas is approved as a drug in the treatment of infants in choking emergencies. A large study is currently testing xenon gas ventilation in adults after cardiac arrest and successful resuscitation. Xenon gas is currently not used for any cancer treatment.

Inhaled xenon gas can cross the blood-brain barrier and accumulates in high concentrations in brain tissue within minutes. There, AMPAR and NMDAR are altered in their function by binding with xenon gas as an antagonist. However, the channels are not completely blocked, but only inhibited in their function (so-called allosteric modulation), which allows a medical application. The potency is determined by the xenon dose and is ultimately limited only by the onset of anesthesia. Serious side effects do not occur with xenon application.

Herein, an experimental model was developed combining xenon application with an established radiobiological investigation methodology. In the subsequent experiments, an increase in radiation treatment efficiency could be shown by application of xenon gas to several glioblastoma cell lines (U87MG and U251) after radiation treatment. This confirmed the hypothesis that xenon gas, as an AMPAR and NMDAR antagonist, influences DNA repair processes in the tumor cell. Of note, it has been shown previously that inhibition of AMPAR and NMDAR signaling in healthy cells does not impair DNA repair processes. Xenon gas application alone without irradiation hardly influenced the survival of glioblastoma cells. However, when xenon gas was administered immediately after irradiation, *i.e.*, in the time window of maximal intracellular DNA repair, significantly more tumor cells died than when irradiated alone. This effect was further enhanced by the addition of memantine as an additional NMDAR antagonist in a synergistic manner. Therefore, the application of xenon gas directly after radiation application advantageously represents a method for radiation sensitization in the therapy of high-grade gliomas.

In this context, inhalation of inert gas mixtures prior to or during the application of ionizing radiation is not considered medically justifiable, since the biologically effective dose (BED) is altered by tissue accumulation of denser gases and thus the radiation effect and side effects are no longer controllable. Thus, the administration period immediately after radiation application for a duration of several hours according to the present invention is the only medically justifiable one for the use of a xenon gas mixture in connection with radiotherapy and advantageously corresponds to the period in which radiation-induced DNA damage in tumor cells is repaired ("vulnerable window"). Here, xenon gas can alter the metabolism of tumor cells, which reduces their DNA repair capacity. Thus, the desired radiation effect in the tumor cell is enhanced and ultimately leads to increased tumor cell death. A combination with another AMPAR and/or NMDAR antagonist can further enhance this effect in a synergistic manner, even if the additional agent alone does not show radiation sensitization (e.g., due to too low accumulation in brain tissue).

Further, xenon gas offers the advantage that it is already approved as a drug for use in anesthesiology and emergency medicine and is considered to have very few side effects as well as being safe to use. It accumulates undiminished in the nerve tissue and its concentration can be directly controlled as a gas mixture during inhalation. Thus, its use in the treatment of high-grade gliomas according to the present invention will advantageously prolong the period before tumor recurrence (so-called progression-free survival) due to more effective destruction of tumor cells.

The present invention describes a new application of xenon gas, as an active substance for inhalation, as radiation sensitizer. Moreover, it teaches the use of a xenon gas mixture alone or in combination with another AMPAR or NMDAR antagonist as a further active ingredient immediately after the application of ionizing radiation to sensitize high-grade gliomas. Overall, the present invention intends to increase the effect and efficiency of radiation therapy on high-grade gliomas by making the tumor cells more sensitive to radiation-induced damage independently of alkylating chemotherapy.

The present invention for the first time demonstrates that xenon gas influences the metabolism of glioma cells, making them more sensitive to radiation-induced damage, by reducing their DNA repair capacity. Furthermore, the present invention identifies the mechanism of action, *i.e.,* that xenon exerts its radio-sensitizing effect through the alteration of NMDAR and AMPAR, wherein activation of these receptors leads to elevation of the intracellular calcium concentration and improved DNA double-strand break (DSB) repair of the irradiated cells, and inhibition by NMDAR/-AMPAR antagonists impairs this process.

The figures show:

Figure 1:
Mean cell survival fractions of CFA with U87MG glioma cells after irradiation and treatment with either cell incubator atmosphere or xenon gas and with or without addition of memantine. The survival fractions are displayed in relation to the sham-treated control. Bars indicate the 95% confidence interval. Asterisks indicate a significant difference in numbers of colonies formed to the control ($p < 0.05$).

Figure 2:
Mean cell survival fractions of CFA with U251 glioma cells after irradiation and treatment with either cell incubator atmosphere or xenon gas and with or without addition of memantine. The survival fractions are displayed in relation to the sham-treated control. Bars indicate the 95% confidence interval. Asterisks indicate a significant difference in numbers of colonies formed to the control ($p < 0.05$).

Figure 3:
Mean cell survival fractions of CFA with HeLa cervical cancer cells after irradiation and treatment with either cell incubator atmosphere or xenon gas. The survival fractions are displayed in relation to the sham-treated control. Bars indicate the 95% confidence interval. Asterisks indicate a significate difference in numbers of colonies formed to the control ($p < 0.05$).

Figure 4:
Immunofluorescence staining of cell lines U87MG, U251, and HeLa with green signal for AMPAR and NMDAR subunits (GluA1 and GluN1) and blue signal for DAPI.

The present invention will be further illustrated by the following example without being limited thereto.

Example

In radiobiological research, colony formation assay (CFA) represents an established method to evaluate the *in-vitro-effects* of radiation treatment (RT) alone or in combination with additional substances on cell survival and reproduction capability. While elements, molecules, or pharmaceutical agents in gaseous form (e.g. oxygen, nitrogen, nitrous oxide, or volatile anesthetics) can alter radiation effects on cells and therefore exhibit modulating properties in CFA, their application is more challenging compared to liquids or solids simply dissolved into the cell culture growth medium.

To provide a method for investigation of modulating properties mediated by gaseous agents in combination with ionizing radiation treatment, a system to combine CFA with gas exposure before, during or after radiation treatment was designed. Subsequently CFA were performed with established glioma cell lines U87MG and U251, as well as cervical cancer-derived cell line HeLa investigating the effect of xenon gas exposure on cell survival after radiation treatment. Expression of AMPAR and NMDAR subunits was tested by immunofluorescence staining.

Methods:

*Membrane-capped T25-flasks*

In a standard experimental setting, T25 flasks contain a certain volume of liquid cell medium and residual air/gas, with a total capacity of 70.0 ml for a single T25 flask (FALCON® technical data sheet; Corning Inc., Corning, USA). Additionally, the volume displaced by a capped T25 (FALCON® 25 cm² Rectangular Canted Neck Cell Culture Flask with Vented Cap; Corning Inc., Corning, USA) was determined via submersion in water to be 96.0 ml. T25 flask caps with an integrated porous membrane (vented caps) allowing for gas exchange but preventing microbial contamination are commercially available. The hydrophobic membrane consists of polytetrafluoroethylene with pores of 0.2 $\mu$m.

*Exposure system*

The system consists of a chamber made from clear acrylic glass panels (thickness 1.0 cm) with inner dimensions of 15.0 × 18.0 × 14.0 cm defining a volume of 3780.0 cm³ and a detachable lid containing a round hole (Ø 3.0 cm). Designed to be used in combination with membrane vented T25 cell culture flasks, up to 15 flasks can be stockpiled into 3 stacks of 5 flasks and are fitted into a polyethylene (PE) bag. Next, the PE bag is placed into the chamber with its open side facing upwards. The open side of the PE bag is then fitted through the hole in the lid and connected to the ending of a flexible silicon tube in a gas-tight manner. The lid is secured, and a vacuum pump (Mityvac® Silverline No. 04010; Lincoln Industrial, St. Louis, USA) is attached to the gas tubing, subsequently removing air until the PE-bag is empty and abuts the stack of T25 flasks. Now, vessels containing prepared gas mixtures can be connected to the gas tubing and the PE-bag can be refilled until its expansion reaches the chamber's inside limits, thus defining an exact volume of the filling. Simple clamping of the gas tubing afterwards preserves the atmosphere inside.

Gas *concentration equation*

With the known dimensions and volumes, the concentration of any involved gas during exposure can be calculated for any volume using the equation

$$\frac{V(flasks) * c(flasks) + V(bag) * c(gas\ mixture)}{V(chamber)}$$

with V(flasks) referring to the total gas volume inside the flasks used, V(chamber) as the volume of the chamber excluding the flasks displacement volume and V(bag) as the difference of these. c(flasks) indicates gas concentrations inside of the flasks prior to exposure, c(gas mixture) represents gas concentrations of the added gas mixture.

*Colony Formation Assay*

*In vitro* colony formation assay (CFA) comparing surviving fractions of irradiated U87MG cells and U251 cells exposed to 50% Xe and/or treated with 25 $\mu$M of memantine to a control group were performed. Additionally, a CFA comparing surviving fractions of irradiated HeLa cells exposed to 50% Xe to a control group was implemented. CFA were carried out in triplets of T25-flasks for each irradiation dose of 0, 2, 4, 6 and 8 Gy in three (U87MG and HeLa) or four (U251) independent repetitions.

*Cell culture*

U87MG and HeLa cells were cultured in Dulbecco's modified eagle's medium (DMEM; U87: Biochrom AG, Berlin, Germany; HeLa: Thermo Fisher Scientific, Waltham, US) and U251 cells were cultured in Gibco Roswell Park Memorial Institute (RPMI) 1640 medium + GlutaMAX™ (Thermo Fisher Scientific, Waltham, US). Cells were stored in an incubator (ambient air with additional 5 % $CO_2$) and split regularly before confluence. In preparation of CFA, cells were dissolved, counted, and distributed into T25-flasks starting with 1000 cells (U87MG), 100 cells (U251), or 200 cells (HeLa) per flask for non-irradiated groups increasing the cell count with irradiation dose to 1000, 2000, 5000, and 10.000 cells (U87MG), 200, 600, 2000, and 5000 cells (U251), or 400, 800, 2000, and 4000 cells (HeLa), respectively. Cells were allowed to attach for 14 to 16 h (U87MG), for 4 h (U251), or for 5,5 h (HeLa) and 108 $\mu$l of either memantine-hydrochloride 0,25 mg/ml (Sigma-Aldrich Corp., St. Louis, USA) or distilled water as placebo were added 60min at U87MG and 90min at U251 and HeLa prior to irradiation, resulting in 25 $\mu$M memantine-hydrochloride per flask.

*Irradiation*

For irradiation, 15 flasks (5 triplets, each for 0, 2, 4, 6, 8 Gy) were placed alongside the margin of eight acrylic glass plates (thickness 1.0 cm) with two plates further added on top to balance secondary electrons. Vertical position to the gantry of a conventional linear accelerator (Elekta Synergy, Elekta AB, Stockholm, Sweden) was allocated by wall-mounted lasers marking the center of the flasks' caps membrane, horizontal position was validated by opening the linear accelerator's light field (32.0 × 32.0 cm) and by positioning the plates centrally inside the field. The triplet from the 0 Gy dose-group was removed immediately after placement (sham-irradiation) and exchanged with dummy T25-flasks filled with water. After delivery of 181.2 MU of 6 MeV photons, equivalent to an effective dose of 2 Gy, the 2 Gy-triplet was removed and replaced by dummy flasks. This process was repeated until a cumulative dose of 8 Gy for the last triplet was reached.

*Gas Exposure*

Gas exposure was performed after the radiation treatment described above. The flasks were exposed to a preformed gas mixture for 60 minutes containing either 75 % Xe, 20 % $O_2$ and 5 % $CO_2$ for the xenon group or a mixture with the 75 % Xe-proportion replaced by nitrogen ($N_2$) for the memantine group and the control group. For our exposure system, this resulted in an atmosphere inside of the flasks of either 50 % Xe, 25 % $N_2$, 20 % $O_2$, and 5 % $CO_2$ for the xenon group or 75% $N_2$, 20 % $O_2$, and 5 % $CO_2$ for the memantine group and control group inside of the flasks. After exposure, the flasks membrane caps were sealed with custom PVC-plugs and stored in a heat cabinet for 24 hours. Finally, the membrane caps' plugs were removed and the flasks were transferred into the 5 % $CO_2$-incubator remaining untouched for 13 days (U87MG), 10 days (U251), or 10 days (HeLa).

*Analysis*

14 days (U87MG), 11 days (U251), or 11 days (HeLa) after radiation treatment and gas exposure, cells were fixed, stained and colonies of more than 50 clonal cells were counted. Plating Efficiency (PE) and Surviving Fraction (SF) were calculated from these numbers. After checking for normal distribution, the independent samples t-test (two-tailed) was used to evaluate significance of the results.

*Immunofluorescence Staining*

Cell lines U87MG, U251, and HeLa were cultured as described above for at least 7 days before staining. Then, cells were counted and transferred onto an 8-well chamber slide. After overnight cultivation and subsequent fixation, primary antibodies (GluA1 and GluN1) as well as sham solution (buffered saline) were applied. Secondary antibodies were added after overnight incubation. Additional DAPI for nucleus staining was applied before assessment with inverted microscopy.

Results:

*Colony Formation Assay*

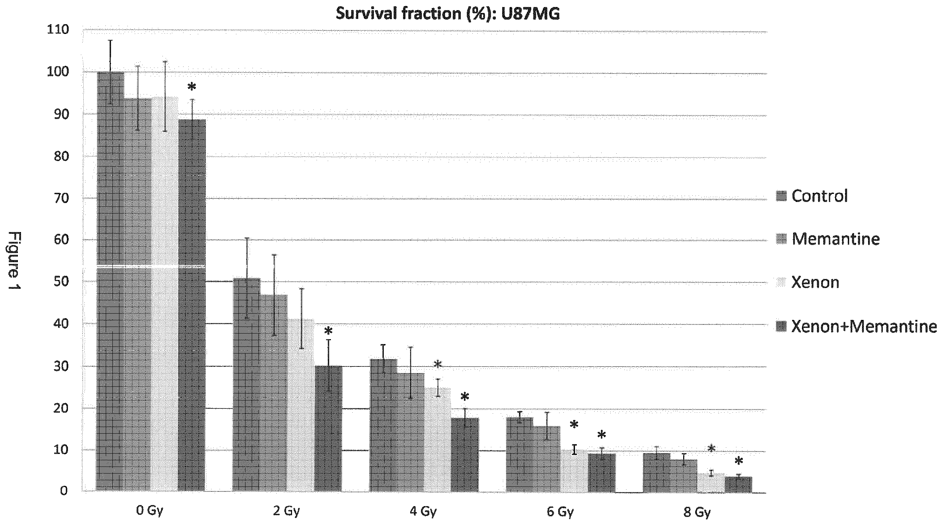

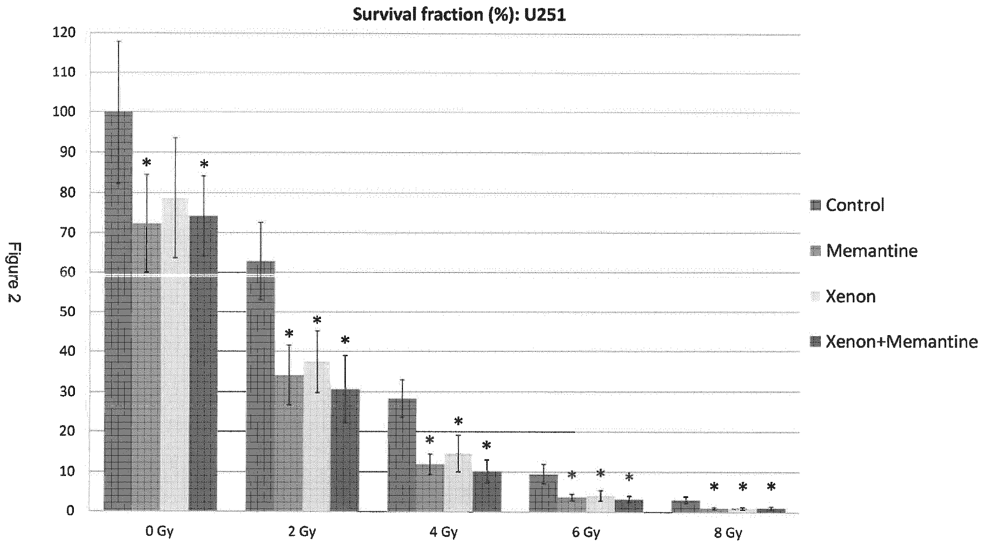

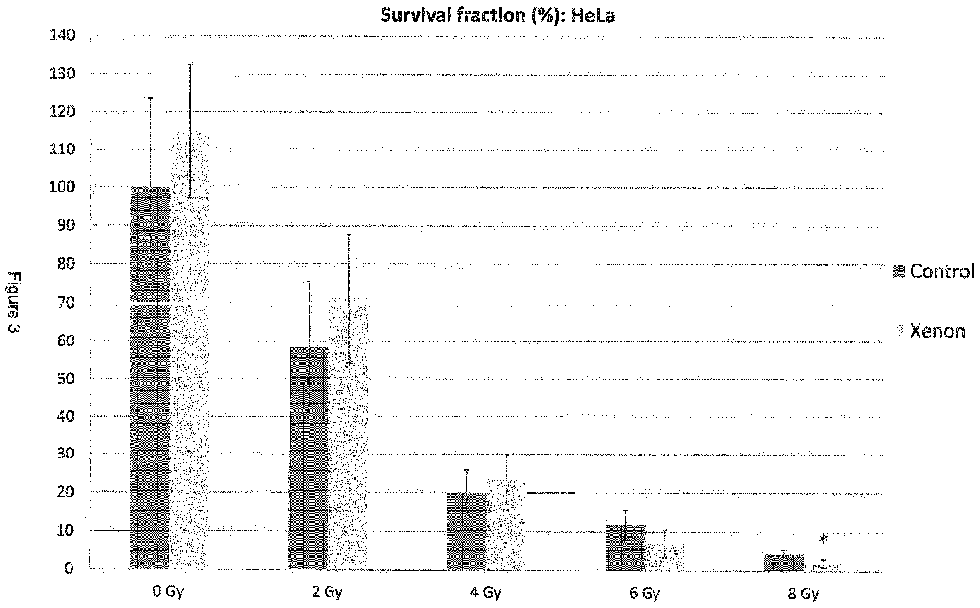

The dose-dependent survival fractions of the CFA with U87MG, U251, and HeLa cells are presented in Figure 1, 2, and 3, respectively.

*U87MG*

For U87MG, mean survival fractions in relation to the unirradiated control group (with 95% CI [lower limit, upper limit]) in the groups neither exposed to xenon nor memantine were 50.9% [41.3, 60.4], 31.9% [28.6, 35.2], 18.0% [16.6, 19.4], and 9.5% [8.0, 11.1] for a radiation dose of 2 Gy, 4 Gy, 6 Gy, and 8 Gy, respectively, with the sham-irradiated group (0 Gy) posing as the control group with 100.0% [92.5, 107.5] survival. After xenon exposure, U87MG mean survival fractions in relation to the control group decreased to 94.2% [86.0, 102.4], 41.5% [34.5, 48.6], 25.0% [22.9, 27.1], 10.3% [9.1, 11.5], and 4.7% [4.0, 5.3] for a radiation dose of 0 Gy, 2 Gy, 4 Gy, 6 Gy, and 8 Gy, respectively, with significant differences in the number of colonies formed to the group not exposed to xenon at 4 Gy (t(16)=3.5, p<.001), 6 Gy (t(16)=8.2, p<.001), and 8 Gy (t(16)=5.7, p<.001), respectively. With added memantine, U87MG mean survival fractions in relation to the control group decreased to 93.8% [86.2, 101.4], 46.8% [37.3, 56.4], 28.5% [22.5, 34.4], 15.9% [12.6, 19.2], and 8.0% [6.7, 9.3] for a radiation dose of 0 Gy, 2 Gy, 4 Gy, 6 Gy, and 8 Gy, respectively, with no significant differences to the groups without added memantine. After xenon exposure and with added memantine, U87MG mean survival fractions in relation to the control group decreased to 88.8% [84.1, 93.5], 30.2% [24.1, 36.3], 17.8% [15.6, 20.1], 9.3% [7.9, 10.7], and 3.9% [3.4, 4.4] for a radiation dose of 0 Gy, 2 Gy, 4 Gy, 6 Gy, and 8 Gy, respectively, with significant differences in the number of colonies formed to the group neither exposed to xenon nor memantine at 0 Gy (t(15)=2.4, p=.03), 2 Gy (t(16)=3.58, p=.003), 4 Gy (t(16)=6.87, p<.001), 6 Gy (t(15)=8.59, p<.001), and 8 Gy (t(16)=6.85, p<.001), respectively.

*U251*

For U251 mean survival fractions in relation to the unirradiated control group in the groups neither exposed to xenon nor memantine were 62.8% [53.1, 72.6], 28.3% [23.6, 32.9], 9.5% [7.1, 12.0], and 3.1% [2.3, 4.0] for a radiation dose of 2 Gy, 4 Gy, 6 Gy, and 8 Gy, respectively, with the sham-irradiated group (0 Gy) posing as the control group with 100.0% [82.1, 117.9] survival. After xenon exposure, U251 mean survival fractions in relation to the control group decreased to 78.7% [63.6, 93.7], 37.5% [29.8, 45.2], 14.6% [10.1, 19.1], 4.2% [2.9, 5.4], and 1.0% [0.6, 1.3] for a radiation dose of 0 Gy, 2 Gy, 4 Gy, 6 Gy, and 8 Gy, respectively, with significant differences in the number of colonies formed to the group not exposed to xenon at 2 Gy (t(20)=4.1, p<.001), 4 Gy (t(21)=4.1, p<.001), 6 Gy (t(22)=3.8, p<.001), and 8 Gy (t(22)=4.9, p<.001), respectively. With added memantine, U251 mean survival fractions in relation to the control group decreased to 72.3% [60.0, 84.5] 34.1% [26.7, 41.5], 11.9% [9.4, 14.5], 3.8% [3.0, 4.6], and 1.0% [0.7, 1.2] for a radiation dose of 0 Gy, 2 Gy, 4 Gy, 6 Gy, and 8 Gy, respectively, with significant differences in the number of colonies formed to the group without added memantine at 0 Gy (t(22)=2.5, p=.02), 2 Gy (t(18)=4.6, p<.001), 4 Gy (t(19)=5.9, p<.001), 6 Gy (t(21)=4.2, p<.001), and 8 Gy (t(22)=4.9, p<.001). After xenon exposure and with added memantine, U251 mean survival fractions in relation to the control group decreased to 74.1% [64.1, 84.1], 30.6% [22.2, 38.9], 10.2% [7.3, 13.1], 3.3% [2.5, 4.1], and 1.0% [0.6, 1.4] for a radiation dose of 0 Gy, 2 Gy, 4 Gy, 6 Gy, and 8 Gy, respectively, with significant differences in the number of colonies formed to the group neither exposed to xenon nor memantine at 0 Gy (t(22)=2.5, p=.02), 2 Gy (t(20)=5.0, p<.001), 4 Gy (t(21)=6.6, p<.001), 6 Gy (t(21)=4.6, p<.001), and 8 Gy (t(21)=4.4, p<.001), respectively.

*HeLa*

For HeLa mean survival fractions in relation to the unirradiated control group in the groups not exposed to xenon were 58.5% [41.3, 75.8], 20.1% [14.1, 26.0], 11.8% [7.9, 15.7], and 4.6% [3.6, 5.6] for a radiation dose of 2 Gy, 4 Gy, 6 Gy, and 8 Gy, respectively, with the sham-irradiated group (0 Gy) posing as the control group with 100.0% [76.5, 123.6] survival. After xenon exposure, HeLa mean survival fractions in relation to the control group increased to 114.7% [97.2, 132.3], 71.1% [54.4, 87.8], 23.5% [17.0, 30.0] and decreased to 7.1% [3.5, 10.7], and 2.0% [1.0, 3.1] for a radiation dose of 0 Gy, 2 Gy, 4 Gy, 6 Gy, and 8 Gy, respectively, with a significant difference in the number of colonies formed to the group not exposed to xenon only at 8 Gy (t(16)=3.4, p=.004).

*AMPAR and NMDAR expression*

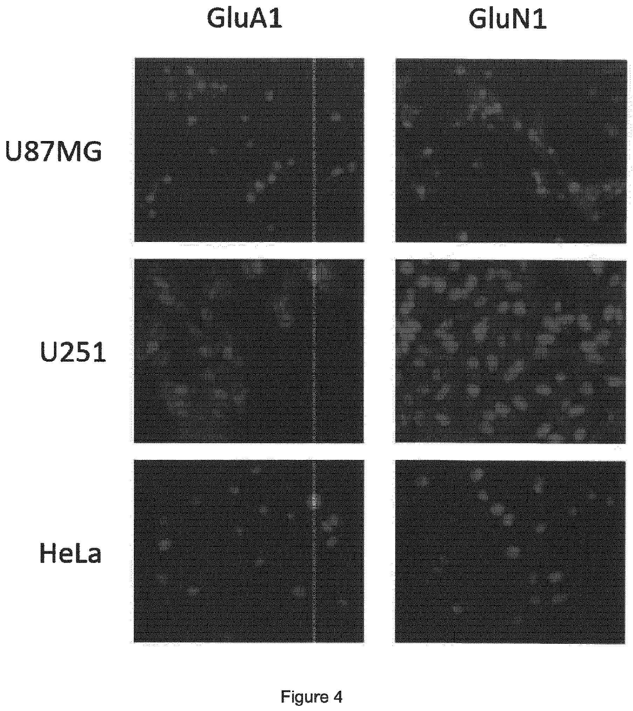

Representative images of the immunofluorescence slides obtained by inverted microscopy are presented in Figure 4. A strong signal could be detected for GluA1 and GluN1 receptor subunits in U87MG and U251. In HeLa, signals for all receptor subunits were weak.

Discussion:

The significant and replicable decrease in dose-dependent survival of U87MG and U251 cells after xenon exposition indicates the impact of a combination treatment with irradiation and subsequent xenon application on U87MG and U251 glioma cells. A decrease in clonogenic survival of U87MG under radiation treatment has been shown before for memantine. As both, xenon and memantine, exhibit NMDAR-modulating properties, their similar and additive effect on dose-dependent survival in U87MG and U251 glioma cells can be explained. Correspondingly, receptor subunits of AMPAR (GluA1) and NMDAR (GluN1) were detected on both cell lines, U87MG and U251. For HeLa cells as non-neuronal derived cancer cells, functional AMPAR or NMDAR have not been reported before and receptor subunits could not be detected by immunofluorescence staining. As hypothesized, xenon exposure after irradiation had no impact on HeLa cell survival except at a very high radiation dose level.

**Claims**

1. Xenon gas for use in an *in vivo* method of sensitizing glioma cells in a subject for radiation therapy, wherein the xenon gas is administered to the subject after said radiation therapy, **characterised in that** the glioma cells are glioma cells having an unmethylated promoter of the $O^6$-methylguanine-DNA methyl-transferase , MGMT, gene.

2. The xenon gas for use according to claim 1, wherein the subject does not undergo concurrent chemotherapy.

3. The xenon gas for use according to claim 1 or claim 2, wherein administration of the xenon gas to the subject starts at the latest 360 minutes after completion of said radiation therapy.

4. The xenon gas for use according to any one of claims 1 to 3, wherein administration of the xenon gas to the subject starts immediately after completion of said radiation therapy.

5. The xenon gas for use according to any one of claims 1 to 4, wherein administration of the xenon gas to the subject continues for at least 30 minutes.

6. The xenon gas for use according to any one of claims 1 to 4, wherein administration of the xenon gas to the subject continues for about 360 minutes.

7. The xenon gas for use according to any one of claims 1 to 6, wherein the xenon gas is administered to the subject by mouth inhalation and/or nasal inhalation.

8. The xenon gas for use according to any one of claims 1 to 7, wherein the xenon gas is administered to the subject in a mixture of xenon gas and at least one further breathable gas.

9. The xenon gas for use according to any one of claims 1 to 8, wherein the radiation therapy uses ionizing radiation.

10. The xenon gas for use according to claim 9, wherein the ionizing radiation is selected from the group consisting of photon beam radiation, electron beam radiation, proton beam radiation, and heavy ion beam radiation.

11. The xenon gas for use according to claim 10, wherein the ionizing radiation is photon beam radiation.

12. The xenon gas for use according to any one of claims 1 to 11, wherein the subject undergoes concurrent therapy with an alpha-amino-3-hydroxy-5-methyl-4-isoxazole propionic acid receptor, AMPAR, antagonist and/or an N-methyl-D-aspartate receptor, NMDAR, antagonist.

13. The xenon gas for use according to claim 12, wherein the AMPAR antagonist and/or NMDAR antagonist is selected from the group, consisting of the AMPAR antagonists talampanel, perampanel, tezampanel, selurampanel, and GYKI-52466; the AMPAR and NMDAR antagonist kaitocephalin; and the NMDAR antagonists memantine, nitromemantine, neramexane, atomoxetine, amantadine, riluzole, dizocilpine, and ifenprodil.

14. The xenon gas for use according to any one of claims 1 to 13, wherein the subject undergoes concurrent therapy with the NMDAR antagonist memantine.

15. A method of sensitizing glioma cells for radiation *in vitro,* comprising the step of administering xenon gas to said glioma cells after said glioma cells have been exposed to said radiation, wherein the glioma cells are glioma cells having an unmethylated promoter of the $O^6$-methylguanine-DNA methyl-transferase, MGMT, gene.

**Patentansprüche**

1. Xenongas zur Verwendung in einem *in vivo* Verfahren zur Sensibilisierung von Gliomzellen in einem Subjekt für eine Strahlentherapie, wobei das Xenongas dem Subjekt nach der Strahlentherapie verabreicht wird, **dadurch gekennzeichnet, dass** die Gliomzellen Gliomzellen sind, die einen unmethylierten Promotor des $O^6$-Methylguanin-DNA-Methyltransferase-, MGMT-, Gens aufweisen.

2. Xenongas zur Verwendung nach Anspruch 1, wobei das Subjekt keiner gleichzeitigen Chemotherapie unterzogen wird.

3. Xenongas zur Verwendung nach Anspruch 1 oder Anspruch 2, wobei die Verabreichung des Xenongases an das Subjekt spätestens 360 Minuten nach Abschluss der Strahlentherapie beginnt.

4. Xenongas zur Verwendung nach einem der Ansprüche 1 bis 3, wobei die Verabreichung des Xenongases an das Subjekt unmittelbar nach Abschluss der Strahlentherapie beginnt.

5. Xenongas zur Verwendung nach einem der Ansprüche 1 bis 4, wobei die Verabreichung des Xenongases an das Subjekt mindestens 30 Minuten lang fortgesetzt wird.

**6.** Xenongas zur Verwendung nach einem der Ansprüche 1 bis 4, wobei die Verabreichung des Xenongases an das Subjekt etwa 360 Minuten lang fortgesetzt wird.

**7.** Xenongas zur Verwendung nach einem der Ansprüche 1 bis 6, wobei das Xenongas dem Subjekt durch orale Inhalation und/oder nasale Inhalation verabreicht wird.

**8.** Xenongas zur Verwendung nach einem der Ansprüche 1 bis 7, wobei das Xenongas dem Subjekt in einer Mischung aus Xenongas und mindestens einem weiteren atembaren Gas verabreicht wird.

**9.** Xenongas zur Verwendung nach einem der Ansprüche 1 bis 8, wobei die Strahlentherapie ionisierende Strahlung verwendet.

**10.** Xenongas zur Verwendung nach Anspruch 9, wobei die ionisierende Strahlung ausgewählt ist aus der Gruppe, bestehend aus Photonenstrahlung, Elektronenstrahlung, Protonenstrahlung und Schwerionenstrahlung.

**11.** Xenongas zur Verwendung nach Anspruch 10, wobei die ionisierende Strahlung Photonenstrahlung ist.

**12.** Xenongas zur Verwendung nach einem der Ansprüche 1 bis 11, wobei das Subjekt einer gleichzeitigen Behandlung mit einem Alpha-Amino-3-hydroxy-5-methyl-4-isoxazolpropionsäure-Rezeptor-, AMPAR-, Antagonisten und/oder einem N-Methyl-D-Aspartat-Rezeptor-, NMDAR-, unterzogen wird.

**13.** Xenongas zur Verwendung nach Anspruch 12, wobei der AMPAR-Antagonist und/oder NMDAR-Antagonist ausgewählt ist aus der Gruppe, bestehend aus den AMPAR-Antagonisten Talampanel, Perampanel, Tezampanel, Selurampanel und GYKI-52466; dem AMPAR- und NMDAR-Antagonisten Kaitocephalin; und den NMDAR-Antagonisten Memantin, Nitromemantin, Neramexan, Atomoxetin, Amantadin, Riluzol, Dizocilpin und Ifenprodil.

**14.** Xenongas zur Verwendung nach einem der Ansprüche 1 bis 13, wobei das Subjekt einer gleichzeitigen Behandlung mit NMDAR-Antagonisten Memantin unterzogen wird.

**15.** Verfahren zur Sensibilisierung von Gliomzellen für Strahlung *in vitro,* umfassend den Schritt des Verabreichens von Xenongas an die Gliomzellen, nachdem die Gliomzellen der Strahlung ausgesetzt wurden, wobei die Gliomzellen Gliomzellen sind, die einen unmethylierten Promotor des $O^6$-Methylguanin-DNA-Methyltransferase-, MGMT-, Gens aufweisen.

## Revendications

**1.** Xénon gazeux destiné à être utilisé dans un procédé *in vivo* de sensibilisation de cellules de gliome chez un sujet pour radiothérapie, dans lequel le xénon gazeux est administré au sujet après ladite radiothérapie, **caractérisé en ce que** les cellules de gliome sont des cellules de gliome ayant un promoteur non méthylé du gène de méthyltransférase de $O^6$-méthylguanine-ADN, MGMT.

**2.** Xénon gazeux destiné à être utilisé selon la revendication 1, dans lequel le sujet ne subit pas de chimiothérapie concomitante.

**3.** Xénon gazeux destiné à être utilisé selon la revendication 1 ou revendication 2, dans lequel l'administration du xénon gazeux au sujet démarre au plus tard 360 minutes après achèvement de ladite radiothérapie.

**4.** Xénon gazeux destiné à être utilisé selon l'une quelconque des revendications 1 à 3, dans lequel l'administration du xénon gazeux au sujet démarre immédiatement après achèvement de ladite radiothérapie.

**5.** Xénon gazeux destiné à être utilisé selon l'une quelconque des revendications 1 à 4, dans lequel l'administration du xénon gazeux au sujet continue pendant au moins 30 minutes.

**6.** Xénon gazeux destiné à être utilisé selon l'une quelconque des revendications 1 à 4, dans lequel l'administration du xénon gazeux au sujet continue pendant au moins 360 minutes.

**7.** Xénon gazeux destiné à être utilisé selon l'une quelconque des revendications 1 à 6, dans lequel le xénon gazeux est administré au sujet par inhalation buccale et/ou inhalation nasale.

8. Xénon gazeux destiné à être utilisé selon l'une quelconque des revendications 1 à 7, dans lequel le xénon gazeux est administré au sujet dans un mélange de xénon gazeux et d'au moins un autre gaz respirable.

9. Xénon gazeux destiné à être utilisé selon l'une quelconque des revendications 1 à 8, dans lequel la radiothérapie utilise un rayonnement ionisant.

10. Xénon gazeux destiné à être utilisé selon la revendication 9, dans lequel le rayonnement ionisant est sélectionné parmi le groupe constitué d'un rayonnement de faisceau de photons, d'un rayonnement de faisceau d'électrons, d'un rayonnement de faisceau de protons et d'un rayonnement de faisceau d'ions lourds.

11. Xénon gazeux destiné à être utilisé selon la revendication 10, dans lequel le rayonnement ionisant est un rayonnement de faisceau de photons.

12. Xénon gazeux destiné à être utilisé selon l'une quelconque des revendications 1 à 11, dans lequel le sujet subit une thérapie concomitante avec un antagoniste du récepteur d'acide alpha-amino-3-hydroxy-5-méthyl-4-isoxazolepropionique, AMPAR et/ou un antagoniste du récepteur de N-méthyl-D-aspartate, NMDAR.

13. Xénon gazeux destiné à être utilisé selon la revendication 12, dans lequel l'antagoniste AMPAR et/ou l'antagoniste NMDAR est sélectionné parmi le groupe constitué des antagonistes AMPAR talampanel, pérampanel, tézampanel, sélurampanel et GYKI-52466; l'antagoniste AMPAR et NMDAR kaïtocéphaline; et les antagonistes NMDAR mémantine, nitromémantine, néramexane, atomoxétine, amantadine, riluzole, dizocilpine et ifenprodil.

14. Xénon gazeux destiné à être utilisé selon l'une quelconque des revendications 1 à 13, dans lequel le sujet subit une thérapie concomitante avec l'antagoniste NMDAR mémantine.

15. Procédé de sensibilisation de cellules de gliome pour rayonnement *in vitro,* comprenant l'étape d'administration de xénon gazeux auxdites cellules de gliome après que lesdites cellules de gliome ont été exposées audit rayonnement, dans lequel les cellules de gliome sont des cellules de gliome ayant un promoteur non méthylé du gène de méthyltransférase de $O^6$-méthylguanine-ADN, MGMT.

Figure 1

Figure 2

Survival fraction (%): HeLa

Figure 3

EP 4 370 202 B1

15

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017341980 A1